# EUROPEAN PATENT SPECIFICATION

(11) **EP 2 974 743 B1**
(45) Date of publication and mention of the grant of the patent: **24.03.2021**
(21) Application number: 13863054.6
(22) Date of filing: 10.12.2013
(51) Int. Cl.: A61K 47/61, A61K 47/40, A61K 31/366, A61P 33/06, A61P 35/00, C08B 37/16, A61K 31/724, A61K 31/365

(54) **CYCLODEXTRIN CARRIER-BASED ARTEANNUIN COMPOUND CONJUGATE AND PREPARING METHOD THEREOF**
CYCLODEXTRINTRÄGERBASIERTES ARTEANNUINVERBINDUNGSKONJUGAT UND HERSTELLUNGSVERFAHREN DAFÜR
CONJUGUÉ DE COMPOSÉ D'ARTÉANNUINE À BASE DE SUPPORT EN CYCLODEXTRINE ET SON PROCÉDÉ DE PRÉPARATION

(30) Priority: 14.12.2012 CN 201210543775
(43) Date of publication of application: 20.01.2016
(73) Proprietor: KPC Pharmaceuticals, Inc., Kunming, Yunnan 650106 (CN)
(72) Inventor: YANG, Bo, Kunming Yunnan 650106 (CN); CHEN, Yunjian, Kunming Yunnan 650106 (CN); YANG, Zhaoxiang, Kunming Yunnan 650106 (CN); ZHU, Ze, Kunming Yunnan 650106 (CN); XIAO, Dan, Kunming Yunnan 650106 (CN); ZHAO, Yulin, Kunming Yunnan 650106 (CN); LIAO, Xiali, Kunming Yunnan 650106 (CN)
(74) Representative: HGF
(86) International application number: PCT/CN2013/088936
(87) International publication number: WO 2014/090127

(56) References cited:
- WO-A2-03/075904
- CN-A- 101 954 090
- CN-A- 102 716 491
- US-A1- 2002 147 177
- UDO K ET AL: "5-Fluorouracil acetic acid/<2>-cyclodextrin conjugates: Drug release behavior in enzymatic and rat cecal media", INTERNATIONAL JOURNAL OF PHARMACEUTICS, ELSEVIER BV, NL, vol. 388, no. 1-2, 30 March 2010 (2010-03-30), pages 95-100, XP026911766, ISSN: 0378-5173, DOI: 10.1016/J.IJPHARM.2009.12.039 [retrieved on 2010-02-17]
- DAVIS ET AL: "Design and development of IT-101, a cyclodextrin-containing polymer conjugate of camptothecin", ADVANCED DRUG DELIVERY REVIEWS, ELSEVIER, AMSTERDAM, NL, vol. 61, no. 13, 12 November 2009 (2009-11-12), pages 1189-1192, XP026698382, ISSN: 0169-409X, DOI: 10.1016/J.ADDR.2009.05.005 [retrieved on 2009-08-12]
- CHALLA R ET AL: "Cyclodextrins in drug delivery: an updated review", AAPS PHARMSCITECH, SPRINGER NEW YORK LLC, US, vol. 6, no. 2, 14 October 2005 (2005-10-14), pages e329-e357, XP008136705, ISSN: 1530-9932, DOI: 10.1208/PT060243
- MARIA P. CRESPO-ORTIZ ET AL: "Antitumor Activity of Artemisinin and Its Derivatives: From a Well-Known Antimalarial Agent to a Potential Anticancer Drug", JOURNAL OF BIOMEDICINE AND BIOTECHNOLOGY, vol. 52, no. 6, 1 January 2012 (2012-01-01), pages 818-18, XP055281483, US ISSN: 1110-7243, DOI: 10.1007/s00262-010-0968-0

## Description

### TECHNICAL FIELD

The present invention relates to the field of pharmaceutical synthesis, and in particular to a cyclodextrin carrier-based arteannuinoid compound conjugate and preparing method thereof.

### BACKGROUND OF THE INVENTION

Artemisinin, a new generation of antimalarial agent as a sesquiterpene lactone compound containing a peroxide group (sesquiterpene 1,2,4-trioxane (a sesquiterpene lactone endope roxide), AMS), was isolated from *Artemisia annua L.* in 1970s, which is effective against *Plasmodium falciparum* resistant to chloroquine. The arteannuinoid compounds, such as artemether, dihydroarteannuin, arteether and artesunate, are effective against *Plasmodium falciparum* which is resistant or sensitive to chloroquine and brain malaria. The arteannuin combination therapy (ACT) recommended by the World Health Organization (WHO), which is rapid and reliable for people infected with malaria, is widely accepted in a majority of countries where malaria is epidemic.

In recent years, it is found through studies that the arteannuinoid compounds exhibit good effect of killing cancer cells. Arteannuin can selectively kill cancer cells with a minimum influence on normal cells. Arteannuin has a mechanism of action distinguishing from traditional chemotherapeutics for tumor, which can reverse multidrug resistance of the tumor cells. The leukemic cell lines resistant to doxorubicin, vincristine, methotrexate or hydroxycarbamide do not have cross resistance to artesunate. Arteannuin has already been used in clinical as an antimalarial agent for many years, and thus its safety has been confirmed in thousands of clinical cases. The antitumor mechanism thereof is still under study, which may be relevant to reaction between arteannuin and Fe²⁺ producing a large amount of free radicals and alkylation, and is also closely related to the induction of tumor cell apoptosis. The concentration of iron ion in cancer cells is much higher than that in normal cells. The peroxide bridge in the structure of arteannuin is cleavaged by iron catalysis, producing free radicals, thereby kill the cells via free radical pathway. In addition, investigations demonstrate that arteannuin may induce apoptosis of mouse leukemic cancer cells and human hepatoma cells. However, the specific mechanism for stimulation (the agent acts on the target site) and effect (cell apoptosis) is still unclear. Investigations further suggest that arteannuin affects, to some extent, the tumor angiogenesis factors, and artesunate has a broad-spectrum antitumor activity.

*In vitro* studies show that, the arteannuinoid agents act on P388 leukemic cells cultured *in vitro,* and significantly inhibit the proliferation of leukemic cells. Some arteannuic acids and arteannuic acid compounds selectively inhibit various cancer cells *in vitro.* Besides, sodium artesunate shows killing effect on human cervical cancer HeLa cells, human poorly differentiated squamous epithelial cell carcinoma of nasopharynx (HeLa, SUNE-1 and CNE2), and significantly inhibits the growth of hepatoma BEL-7402 cells and xenografted human hepatoma cells in naked mice. Artesunate can induce apoptosis of U937 cells, and has killing effect on human colon cancer HCT-8 cells, human erythroleukemia K562 cells and human breast cancer cell strain MCF-7.

*In vivo* experiments: it was found in the experimental studies on the effect of different doses of artesunate on mouse Hep2 hepatoma that, 60 mg/kg of artesunate via intraperitoneal injection significantly inhibits mouse Hep2 hepatoma, with an anti-tumor rate of 80.4%. It was found that artesunate significantly inhibits mouse hepatoma and S180 solid tumor via administration mode of intramuscular injection. It was found thrgough the experiments on the inhibition effect of artesunate on mouse hepatocarcinoma H22, similar to positive control 5-Fu, when 300 mg/kg of artesunate is administrate by gavage, the anti-tumor rate is above 40%. The growth of transplanted fibrosarcoma of an experimental mouse may be significantly inhibited after oral administration of ferrous sulfate then 6 hours later by administration of dihydroarteannuin, and the effect is greatly enhanced compared with dihydroarteannuin alone. In addition, arteannuinoid agents may also inhibit growth of tumors such as human ovarian cancer HO-8910 transplanted tumor in naked mice and prostatic cancer in transgenic mice.

However, the arteannuinoid agents have low solubility in water, low bioavailability, and cannot be favorably transported onto a cancer cell. In addition, they are eliminated rapidly in human and animal models, and thus cannot reach therapeutic dose at the surface of cancer cells.

Cyclodextrins (CDs) are semi-natural macromolecular compounds produced from starch by means of enzymatic digestion of cyclodextrin glucosyltransferase. It can hardly be hydrolyzed, and is merely absorbed in a small amount when passing through stomach and small intestine of a human body. Cyclodextrins may be fermented by microorganisms in the colon and degraded into monosaccharide or disaccharide, and thus can be adsorbed by large intestine.

WO 03/075904 is directed to a stable form of artemisinin wherein an artelinic acid or artesunic acid is complexed with cyclodextrin analogs, preferably, beta-cyclodextrin. The complexed cyclodextrin artemisinin formulation shields the peroxide portion of the artemisinin backbone from hydrolytic decomposition rendering it stable in solution. Artelinic acid and cyclodextrin are placed into contact with one another to yield a 2:1 molecular species. Artesunic acid and cyclodextrin yield a 1:1 molecular species. The complexed cyclodextrin artemisinin formulation is effective for the treatment of malaria and is stable in solution for long periods of time.

The present inventors surprisingly found from investigations that, the cyclodextrin-based arteannuinoid compound conjugate can be prepared by using cyclodextrin as a carrier. It is expected to overcome the above defects of the arteannuinoind agents, improve the therapeutic effect and reduce toxic or side effects.

### SUMMARY OF THE INVENTION

Based on the above discovery, one of the objects of the present invention is to provide a targeted cyclodextrin carrier-based arteannuinoid compound conjugate. The invention is defined by the independent claims. Further optional features of the invention are defined by the dependent claims.

Those skilled in the art would appreciate that, "conjugate" refers to a new molecule entity formed by connecting two or more molecules via a covalent bond therebetween. In the present disclosure, the cyclodextrin carrier-based arteannuinoid compound conjugate refers to a molecule formed by connecting an arteannuinoid compound with a cyclodextrin compound via an ester bond; in particular and according to the present invention, to a molecule formed by the ester bond formed between a carboxyl group of the arteannuinoid compound and a hydroxyl group of the cyclodextrin compound .

In order to achieve the above object, the present disclosure adopts the following technical solutions:

A cyclodextrin carrier-based arteannuinoid compound conjugate is provided, wherein the arteannuinoid compound is connected by using an ester bond formed between a carboxyl group thereof and a hydroxyl group of the cyclodextrin. The specific position of the hydroxyl group used to form the ester bond is not defined, which can be any one of the hydroxyl groups in the cyclodextrin molecule.

The arteannuinoid compounds are a class of arteannuin-based compounds containing peroxide bridge, including a compound formed by modifying the structure of the arteannuin molecule. The preferred arteannuinoid compounds of the present disclosure include such as artesunate, a compound containing a phenoxyl group at C-12 position of arteannuin, a compound containing an alkoxyl group at C-12 position of arteannuin. The particularly preferred compounds are arteannuin, dihydroarteannuin and artesunate. The arteannuinoid compound according to the present invention is a compound of formula II according to the appended claims.

The natural cyclodextrin (abbreviated to CD) is a generic term for a series of cyclic oligosaccharide produced by amylose with the action of cyclodextrin glucosyltransferase generated by bacillus. Among them, molecules containing 6, 7 or 8 glucose units, which are known as α-, β-, and γ-cyclodextrin respectively, attract much more attention and are of great practical value. Based on results from X-ray crystal diffraction, infrared spectrum and nuclear magnetic resonance spectroscopy, it is determined that each D(+)-glucopyranose constituting cyclodextrin has a chair conformation, and each glucose unit forms a cycle with 1,4-glycosidic bond. Cyclodextrin is a hollow cylinder steric loop structure with two open ends, wherein one big and the other small, since the glycosidic bond connecting the glucose units cannot rotate freely. A hydrophobic region is formed in the hollow structure due to shielding effect caused by the C-H bond in the cavity. All the hydroxyl groups locate at the external of the molecule. The end with large opening is composed of C₂ and C₃ secondary hydroxyl groups, and the end with small opening is composed of C₆ primary hydroxyl group, which is highly hydrophilic, and the cyclodextrin has the structure of: 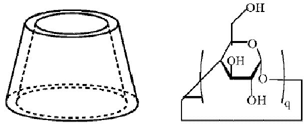 wherein, the compound is α-cyclodextrin when q=6, β-cyclodextrin when q=7 and γ-cyclodextrin when q=8.

Pharmacological studies have shown that cyclodextrin is rarely directly absorbed in human and animal gastrointestinal tract, which is mainly decomposed into open-chained maltodextrin, maltose and glucose by microorganisms in the colon tract, then absorbed by the large intestine. Since cyclodextrin has such unique biological properties, it can be used as a carrier for transporting a drug targeting rectum, thereby to improve the bioavailability of the drug at colon and exert its function.

Cyclodextrin used in the present invention is a sulfonate of a structure shown as formula I: 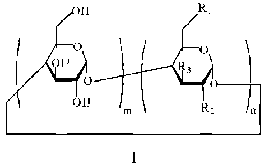
wherein n is 1, and m+n = any one of 6, 7 or 8;
R₁, R₂ or R₃ is -ROH,
wherein R is (CH₂)ₓ, NH(CH₂)ₓ, NH(CH₂)ₓNH(CH₂)ₓ, CO(CH₂)ₓ or O(CH₂)ₓ, x is 0, 1, 2, 3 or 4.

Sulfonated cyclodextrin can be synthesized from cyclodextrin by sulfonylating reaction, then be reacted with the arteannuinoid compound to form an ester bond so as to obtain the cyclodextrin carrier-based arteannuinoid compound.

The synthesis of sulfonyl-modified cyclodextrin may be carried out as descried in the prior art documents. For example, the cyclodextrin is first reacted with a sulfonylation agent to produce sulfonated cyclodextrin [R.C. Petter, J.S. Salek, C.T. Sikorski, G. Kumaravel, and F.-T. Lin: J. Am. Chem. Soc. 112, 3860-3868 (1990)]. The sulfonylation of the cyclodextrin may take place at 2-, 3- and/or 6-position of the D(+)-glucopyranose. For example, a chemical equation showing that the cyclodextrin is modified by mono-substituted p-toluenesulfonyl is as follows:

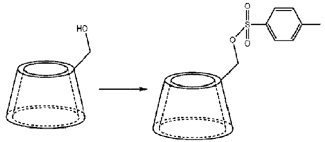

The arteannuinoid compound according to the present disclosure is a compound formed by substitution of the C₁₂ position of the arteannuin molecule with a compound containing a carboxyl group. The synthesis of the arteannuinoid compound may be carried out as descried in the prior art documents.

The dihydroarteannuin may be reacted with a compound containing a carboxyl group to produce an arteannuinoid compound [P. M. O' Neill, et al.: J. Med. Chem. 44, 58-68(2001)]. The dihydrarteannuin may be etherified or esterified at the hydroxyl group of C₁₂ position. The etherifying or esterifying reagent may be various compounds containing a carboxyl group and a hydroxyl group, and the chemical reaction is as follows: 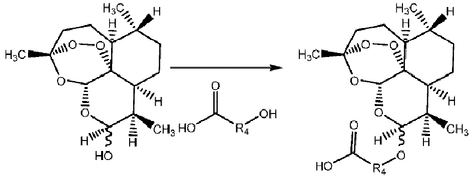 wherein, according to the invention, the arteannuinoid compound has a structure of formula II, 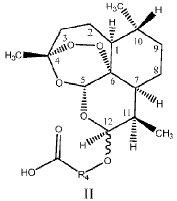 wherein R₄ is (CH₂)_{y}, CO(CH₂)_{y}, C₆H₆(CH₂)_{y} or COC₆H₆(CH₂)_{y}, wherein y is a integer greater than or equal to 0, CO is a carbonyl, and C₆H₆ is a benzene ring.

More preferably, the etherifying or esterifying reagent is selected from the group consisting of succinic acid, succinic anhydride, terephthalic acid, p-hydroxybenzoic acid, p-hydroxybenzoate ester, p-halobenzoic acid and p-halobenzoate, wherein, the "halo" includes "fluoro", "chloro", "bromo" and "iodo".

Another object of the present disclosure is to provide a method for preparing the cyclodextrin carrier-based arteannuinoid compound conjugate of the prevent disclosure.

The arteannuinoid compound has a low solubility in water and oil, and is formed to salts under weak base condition of sodium carbonate, sodium bicarbonate, phosphate salt and the like, producing a water-soluble salt of the arteannuinoid compound.

More preferably, the arteannuinoid compound can be fully dissolved in a sodium bicarbonate solution of 5% to 10%.

In an embodiment of the present disclosure, the salt of the arteannuinoid compound and the sulfonated cyclodextrin are dissolved in a polar organic solvent in a molar ratio of 10:1 to 1:1.5 or in a weight ratio of 1:5 to 1:50.

Preferably, the polar organic solvent is N,N-dimethylformamide, N,N-diethylamide or dimethyl sulfoxide.

The reaction system is stirred at 80 to 120°C for 24 to 192 hours.

The liquid concentrate is dropped into acetone in a weight ratio of 1:5 to 1:50, the resulting suspension is filtered or centrifuged by centrifuge, washed with acetone for 2 to 6 times. The resulting solid is dried, thereby to obtain a crude product of the cyclodextrin carrier-based arteannuinoid compound of the present disclosure.

Preferably, the crude product of the cyclodextrin carrier-based arteannuinoid compound may be dissolved in water or slightly acidic water (pH=6 to 7) in a weight ratio of 1:5 to 1:50, filtered after stirred to mix. The resulting solid is dried in vacuo at 40 to 60°C for 4 to 40 hours, thereby to obtain a pure product of the cyclodextrin carrier-based arteannuinoid compound.

The sulfonated cyclodextrin and the arteannuinoid compound have a low water-solubility, and almost insoluble in water. The inventors have surprisingly found the problem that the arteannuinoid compound is almost insoluble in water can be well solved by connecting the arteannuinoid compound with the sulfonated cyclodextrin via an ester bond.

The above crude product of the cyclodextrin carrier-based arteannuinoid compound conjugate can be purified. The purification includes: drying the reactant solution obtained from the esterifying reaction, collecting the solid residue, dissolving it in water, then, filtering to remove impurity, isolating by organic solvent precipitation method, thus obtaining the pure product of the cyclodextrin carrier-based arteannuinoid compound. The purpose of filtration is to remove the unreacted arteannuinoid compound and the excess sulfonated cyclodextrin.

The pure product of the cyclodextrin carrier-based arteannuinoid compound can be produced by the organic solvent precipitation method, i.e., by adding organic solvent to precipitate the desired product, thereby allowing isolating it. The isolation thereof is simple and efficient.

Preferably, the organic solvent is acetone, methanol, ethanol, isopropanol, chloroform or tetrahydrofuran.

The organic solvent precipitation method is affected by the concentration of the solution. If the concentration of solution is too low, the cyclodextrin carrier-based arteannuinoid compound does not sufficiently precipitate out, thus the recovery rate is low; the solution with a high concentration can save the organic solvent. Therefore, the preparing method according to the present disclosure further preferably comprises a concentration step in order to reduce water in solution, improve the concentration of the solution, thereby to fully precipitate the cyclodextrin carrier-based arteannuinoid compound in the organic solvent.

The crude product of the cyclodextrin carrier-based arteannuinoid compound conjugate can be dissolved in water or slightly acidic water (pH=6 to 7), stirred to mix, filtrated to remove the unreacted arteannuinoid compound.

The preparing method according to the present disclosure further comprises a step of refining the cyclodextrin carrier-based arteannuinoid compound conjugate.

Preferably, the refining is organic solvent extraction or chromatography. The organic solvent is acetone, ether, chloroform or tetrahydrofuran.

In a specific embodiment according to the present disclosure, the structure of mono-6-artesunate-β-cyclodextrin conjugate is determined by NMR and high-resolution mass spectrometry. The ¹HNMR spectrum of mono-6-artesunate-β-cyclodextrin conjugate shows that, under the presence of D₂O, characteristic peaks of H₅, H₁₂, H₁₃, H₁₄ and H₁₅ of the arteannuinoid compound occur at 0.3 to 3.0 ppm and 5.0 to 7.1 ppm, while cyclodextrin does not have a characteristic absorption here. In addition, the artesunate is almost insoluble in water, which can initially indicate that the artesunate has reacted with cyclodextrin. In addition, high resolution mass spectrometry shows that *m*/*z*: 1500.5356(M⁺), *mlz*: 1522.5349(M+Na)⁺.

### Advantageous Effects

The cyclodextrin carrier-based arteannuinoid compound conjugate provided by the present disclosure has a better water-solubility, compared with a compound which does not bond with cyclodextrin. The cyclodextrin carrier-based arteannuinoid compound conjugate according to the present disclosure has a solubility of 45 to 80 mg/ml in water under 25 °C. However, the artemistinin compound which does not bond with cyclodextrin has a lower solubility. Also, the cyclodextrin carrier-based arteannuinoid compound is absorbed in merely a small amount when passing through stomach and small intestine of a human body, which is mainly absorbed by the large intestine, with a property of releasing in straight cecum. Therefore, the cyclodextrin carrier-based arteannuinoid compound according to the present disclosure can effectively enter the body of a patient, improving the bioavailability.

In another aspect, the cyclodextrin carrier-based arteannuinoid compound conjugate according to the present disclosure has anticancer target feature, especially for the target colorectal cancer cells. It is absorbed in merely a small amount when passing through stomach and small intestine of a human body, and decomposed and released in the colon, and then absorbed by the large intestine, and thus has a property of releasing in the colon. The cyclodextrin carrier-based arteannuinoid compound conjugate according to the present disclosure has anticancer target feature, especially for colorectal cancer cells. The cyclodextrin carrier-based arteannuinoid compound conjugate can effectively enter the body of a patient, preferably gather around target cancer cells, and selectively induce rectum cancer cells of a human body to die, thereby improving efficacy and reducing toxic or side effects.

For example, 180 µl suspension of human rectal cancer cells HCT116, Lovo, SW480 and HT29 (at a concentration of 4×10⁴/mL) was added to a 96 well plate, and pre-cultured under 5% CO₂, 37°C and saturated moisture for 24h. 150 µl medium was replaced, and 20 µl medium containing molecular assembly at different concentrations was added. The cells were cultured for another 48h, with the medium free of a sample as a negative control, and a medium containing camptothecin, oxaliplatin or fluorouracil at different concentrations as positive controls. MTT assay was used to determine the OD value, and the growth inhibition rate was calculated. The inhibition of compounds at high, medium and low concentration on the tumor cell proliferation was observed. All the experiments above were performed in triplet. The results of *in vitro* experiment of the arteannuin cyclodextrin conjugate on human rectum and caecum cancer cell are shown in table 1.

**Table 1. The comparison of IC₅₀ values (µM) for the cyclodextrin carrier-based arteannuinoid compound conjugate according to the present disclosure and some arteannuinoid compounds in several rectal cancer cells.**

| (HCT116, Lovo, SW480 and HT 90 Cells | | | | | |
|---|---|---|---|---|---|
| Compd. | MW | IC₅₀(µM) | | | |
| | | HCT116 | Lovo | SW480 | HT 90 |
| oxaliplatin | 397 | 0.76 | 1.51 | 30.1 | 9.7 |
| fluorouracil | 130 | 5.4 | 23.0 | 30.8 | 38.5 |
| dihydroarteannuin | 284 | 1.76 | 2.11 | 14.1 | 21.1 |
| artesunate | 384 | 1.04 | 2.60 | 1.82 | 9.11 |
| 6-artesunate bonded to β-cyclodextrin | 1499 | 0.84 | 0.72 | 3.92 | 5. 30 |

As can be seen from table 1, an arteannuin cyclodextrin conjugate with suitable chain length (such as, 6-artesunate bonded to β-cyclodextrin) is designed and synthesized, which has anticancer activity substantively better than most of the materials without bonding (dihydroarteannuin and artesunate) and the control medicines used in clinical (oxaliplatin and fluorouracil). Without restriction to any theory, the inventor proposes that the arteannuin cyclodextrin conjugate with suitable chain length can preferably gather around target rectum and caecum cancer cells, and selectively induce rectum cancer cells of a human body to die. Since cyclodextrin can be decomposed into open chained maltodextrin, maltose and glucose by microorganisms in the colon tract, and then absorbed by the large intestine, it can be used as a carrier for transporting a drug targeting rectum, thereby to improve the bioavailability of the drug at colon and exert its function. Therefore, the cyclodextrin carrier-based arteannuinoid compound conjugate according to the present disclosure can achieve anticancer target feature, especially for colorectal cancer cells. The cyclodextrin carrier-based artcannuinoid compound conjugate can effectively enter the body of a patient, preferably gather around the target cancer cells, and selectively induce rectum cancer cells of a human body to die, thereby improving efficacy and reducing toxic or side effects.

The method for preparing the cyclodextrin carrier-based arteannuinoid compound conjugate according to the present disclosure is simple to operate, easily available in respect to materials and moderate in respect to reaction conditions, which can be used for manufacturing a great amount of the cyclodextrin carrier-based arteannuinoid compound conjugate according to the present disclosure.

### BRIEF DESCRIPTION OF THE DRAWINGS

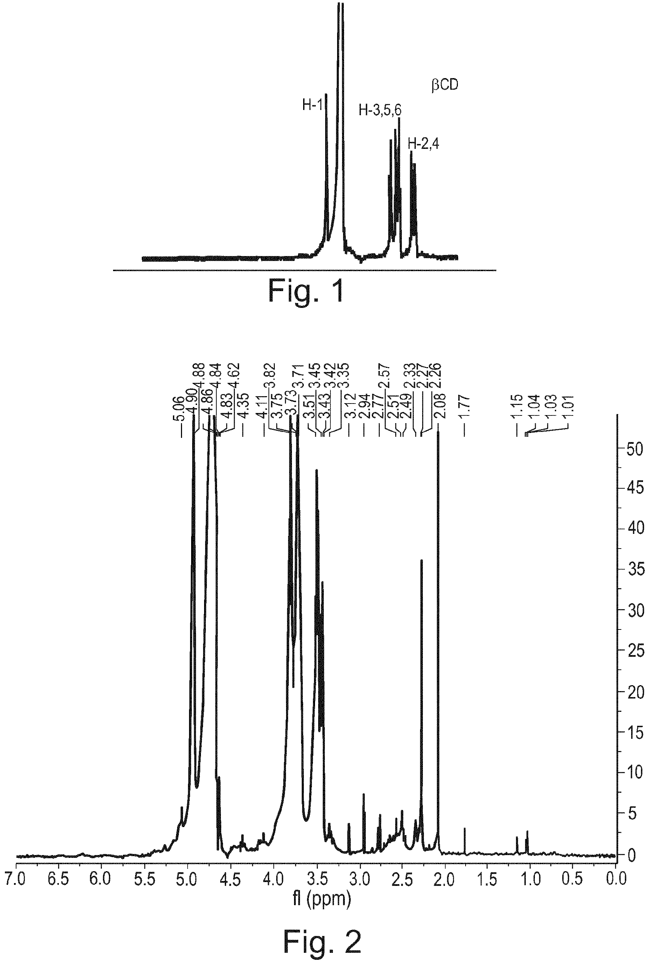

FIG. 1 shows the ¹HNMR spectrum of β-cyclodextrin;
FIG. 2 shows the ¹HNMR spectrum of 6-artesunate bonded β-cyclodextrin conjugate prepared in example 1, which has the characteristic peaks of H₅, H₁₂, H₁₃, H₁₄ and H₁₅ of the arteannuinoid compound at 7 to 8 ppm, however, cyclodextrin does not have the characteristic peak here;

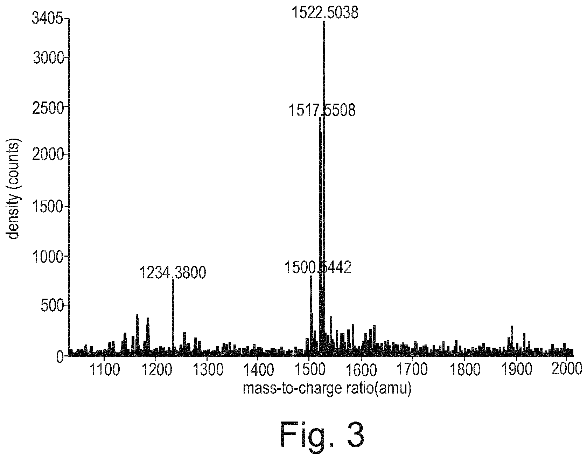

FIG. 3 shows the high resolution mass spectrum [MS(TOF-ESI)] of 6-artesunate bonded β-cyclodextrin conjugate prepared in example 1.

### DETAILED DESCRIPTION

The examples of the present invention disclose a cyclodextrin carrier-based arteannuinoid compound conjugate and the preparing method thereof. Those skilled in the art can modify appropriately the parameters of the process according to the disclosure. The product and method of the present disclosure have been described by the description, particularly the examples.

The present invention will be illustrated in detail in combination with examples in order to further understand the present invention.

### Example1 :

Sodium artesunate (0.512 g, 0.01 mmol) and mono-6-p-toluenesulfonyl -β-cyclodextrin (1.22 g, 0.01 mmol) were added to a 100 ml round bottom flask. N,N-dimethyl sulfoxide (3.0 ml) was added to the flask, and stirred for 24 h at 100°C. The resultant solution was evaporated to dryness under reduced pressure at 60°C. The residue was dissolved in a small amount of water, then, dropped into acetone (300 mL). The resulting precipitate was filtered and dried. Then, the precipitate was dissolved in slightly acidic water (pH = 6), filtered after stirred to mix. The resulting solid was fully dissolved in water, filtered, concentrated, dropped into chloroform (100 mL) to extract and filtered. The precipitate was collected and dried for 24 h under vacuum at 50°C, to obtain mono-6-artesunate-β-cyclodextrin conjugate (yield: about 56%, solubility: 65 mg/mL).

The structure of mono-6-artesunate-β-cyclodextrin conjugate was determined by NMR and high-resolution mass spectrum. The ¹HNMR spectrum of mono-6-artesunate-β-cyclodextrin conjugate shows that, under the presence of D₂O, the characteristic peaks of H₅, H₁₂, H₁₃, H₁₄ and H₁₅ of the arteannuinoid compound occur at 0.3 to 3.0 ppm and 5.0 to 7.1 ppm, while cyclodextrin does not have such characteristic absorption here. In addition, artesunate is almost insoluble in water, which can preliminarily indicate that artesunate has reacted with cyclodextrin. High-resolution mass spectrum shows that *m*/*z*: 1500.5356 (M⁺), *m*/*z*: 1522.5349 (M+Na).

### Example 2:

Sodium artesunate (5.12 g, 0.1 mmol) and mono-2-p-toluenesulfonyl-α-cyclodextrin (1.12 g, 0.01 mmol) were added to a 100 ml round bottom flask. N,N-diethylamide (3.0 ml) was added to the flask, and stirred for 192 h at 80°C. The resultant solution was evaporated to dryness under reduced pressure at 60°C. The residue was dissolved in a small amount of water, then dropped in acetone (300 mL). The resultant precipitate was filtered and dried. Then, the precipitate was dissolved in slightly acidic water (pH = 6), filtered after stirred to mix. The resultant solid was fully dissolved in water, filtered, concentrated, dropped in ether (100 mL) to extract and filtered. The precipitate was collected and dried for 24 h under vacuum at 50°C, to obtain mono-2-artesunate-α-cyclodextrin conjugate (yield: about 60%, solubility: 78 mg/mL).

### Example 3:

Sodium artesunate (0.512 g, 0.01 mmol) and 2,3-p-toluenesulfonyl-γ-cyclodextrin (19.8 g, 0.15 mmol) were added to a 100 ml round bottom flask. Dimethyl sulfoxide (3.0 ml) was added to the flask, and stirred for 96 h at 120°C. The resultant solution was evaporated to dryness under reduced pressure at 60°C. The residue was dissolved in a small amount of water, then dropped in acetone (300 mL). The resultant precipitate was filtered and dried. Then, the precipitate was dissolved in slightly acidic water (pH = 6), filtered after stirred to mix. The resultant solid was fully dissolved in water, filtered, concentrated, and subjected to column chromatography using water as an eluting reagent. The elute solution was collected from the chromatograph and concentrated, then dried for 24 h under vacuum at 50°C, to obtain 2,3-artesunate-γ-cyclodextrin conjugate (yield: about 54%, solubility: 80 mg/mL).

### Example 4:

The compound sodium artesunate having a phenoxy group at C-12 of arteannuin (0.712 g, 0.01 mmol) and mono-6-p-toluenesulfonyl-β-cyclodextrin (1.22 g, 0.01 mmol) were added to a 100 ml round bottom flask. N,N-dimethyl sulfoxide (3.0 ml) was added to the flask, and stirred for 24 h at 100°C. The resultant solution was evaporated to dryness under reduced pressure at 60°C. The residue was dissolved in a small amount of water, then dropped in acetone (300 mL). The resultant precipitate was filtered and dried. Then, the precipitate was dissolved in slightly acidic water (pH = 6), filtered after stirred to mix. The resultant solid was fully dissolved in water, filtered, concentrated, dropped in chloroform (100 mL) to extract and filtered. The precipitate was collected and dried for 24 h under vacuum at 50°C, to obtain a mono-6-arteannuin-β-cyclodextrin conjugate wherein the arteannuin having a phenoxy group at C-12 (yield: about 56%, solubility: 45 mg/mL).

### Example 5:

Sodium artesunate having a C-12 of arteannuin connected with a phenoxy group (7.12 g, 0.1 mmol) and mono-2-p-toluenesulfonyl-α-cyclodextrin (1.12 g, 0.01 mmol) were added to 100 ml round bottom flask. N,N-diethylamide (3.0 ml) was added to flask, and stirred for 192 h at 80°C. The resultant solution was evaporated to dryness under reduced pressure at 60°C. The residue was dissolved in a small amount of water, then, dropped in acetone (300 mL). The resulting precipitate was filtered and dried. Then, the precipitate was dissolved in slightly acidic water (pH = 6), filtered after stirred to mix. The resulting solid was fully dissolved in water, filtered, concentrated, dropped in ether (100 mL) to extract and filtered. The precipitate was collected and dried for 24 h under vacuum at 50°C, thereby to obtain mono-2-arteannuin having a C-12 connected with the phenoxy group-α-cyclodextrin conjugate (yield: about 60%, solubility: 48 mg/mL).

### Example 6:

A compound of arteannuin having a phenoxy group at C12 (0.712 g, 0.01 mmol) and 2,3-p-toluenesulfonyl-γ-cyclodextrin (19.8 g, 0.15 mmol) were added to a 100 ml round bottom flask. Dimethyl sulfoxide (3.0 ml) was added to the flask, and stirred for 96 h at 120°C. The resultant solution was evaporated to dryness under reduced pressure at 60°C. The residue was dissolved in a small amount of water, then dropped in acetone (300 mL). The resultant precipitate was filtered and dried. Then, the precipitate was dissolved in slightly acidic water (pH = 6), filtered after stirred to mix. The resultant solid was fully dissolved in water, filtered, concentrated, and subjected to column chromatography using water as an eluting reagent. The solution obtained from the chromatograph was collected and concentrated, then dried for 24 h under vacuum at 50°C, to obtain a 2,3-arteannuin-γ-cyclodextrin conjugate wherein the arteannuin has a phenoxy group at C-12 (yield: about 54%, solubility: 49 mg/mL).

### Example 7:

Sodium artesunate having an alkoxy group at C-12 of arteannuin (0.612 g, 0.01 mmol) and mono-6-p-toluenesulfonyl-β-cyclodextrin (1.22 g, 0.01 mmol) were added to a 100 ml round bottom flask. N,N-dimethyl sulfoxide (3.0 ml) was added to the flask, and stirred for 24 h at 100°C. The resultant solution was evaporated to dryness under reduced pressure at 60°C. The residue was dissolved in a small amount of water, then dropped in acetone (300 mL). The resultant precipitate was filtered and dried. Then the precipitate was dissolved in slightly acidic water (pH = 6), filtered after stirred to mix. The resultant solid was fully dissolved in water, filtered, concentrated, dropped in chloroform (100 mL) to extract and filtered. The precipitate was collected and dried for 24 h under vacuum at 50°C, to obtain a mono-6-arteannuin-β-cyclodextrin conjugate wherein the arteannuin has an alkoxy group at C-12 (yield: about 56%, solubility: 52 mg/mL).

### Example 8:

Sodium artesunate having an alkoxy group at C-12 (6.12 g, 0.1 mmol) and mono-2-p-toluenesulfonyl-α-cyclodextrin (1.12 g, 0.01 mmol) were added to a 100 ml round bottom flask. N,N-diethylamide (3.0 ml) was added to the flask, and stirred for 192 h at 80°C. The resultant solution was evaporated to dryness under reduced pressure at 60°C. The residue was dissolved in a small amount of water, then dropped in acetone (300 mL). The resultant precipitate was filtered and dried. Then, the precipitate was dissolved in slightly acidic water

(pH = 6), filtered after stirred to mix. The resultant solid was fully dissolved in water, filtered, concentrated, dropped in ether (100 mL) to extract and filtered. The precipitate was collected and dried for 24 h under vacuum at 50°C, to obtain a mono-2-arteannuin-α-cyclodextrin conjugate having an alkoxy group at C-12 (yield: about 60%, solubility: 55 mg/mL).

### Example 9:

A compound of arteannuin having a C12 connected with an alkoxy group (0.612 g, 0.01 mmol) and 2,3-p-toluenesulfonyl-γ-cyclodextrin (19.8 g, 0.15 mmol) were added to 100 ml round bottom flask. Dimethyl sulfoxide (3.0 ml) was added to flask, and stirred for 96 h at 120°C. The resultant solution was evaporated to dryness under reduced pressure at 60°C. The residue was dissolved in a small amount of water, then, dropped in acetone (300 mL). The resulting precipitate was filtered and dried. Then, the precipitate was dissolved in slightly acidic water (pH = 6), filtered after stirred to mix. The resulting solid was fully dissolved in water, filtered, concentrated, and performed with column chromatography using water as an eluting reagent. The solution obtained from the chromatograph was collected and concentrated, then dried for 24 h under vacuum at 50 °C , to obtain a 2,3-arteannuin-γ-cyclodextrin conjugate wherein arteannuin has an alkoxy group at C-12 (yield: about 54%, solubility: 56 mg/mL).

The above examples are provided to illustrate the method according to the present invention and the core concept thereof.

## Claims

1. A cyclodextrin carrier-based arteannuinoid compound conjugate, wherein the arteannuinoid compound is connected through an ester bond formed between a carboxyl group of the arteannuinoid compound and a hydroxyl group of the cyclodextrin;
wherein the cyclodextrin is a sulfonate of a structure shown as formula I: 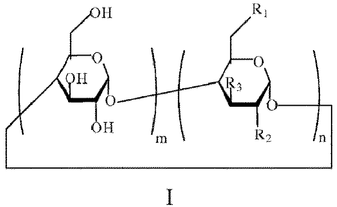
wherein n is 1, and m+n = any one of 6, 7 or 8;
R₁, R₂ or R₃ is -ROH;
wherein R is -(CH₂)ₓ, -NH(CH₂)ₓ, -NH(CH₂)ₓNH(CH₂)ₓ, -CO(CH₂)ₓ or -O(CH₂)ₓ, and x is 0, 1, 2, 3 or 4;
wherein the arteannuinoid compound has a structure shown as formula II, 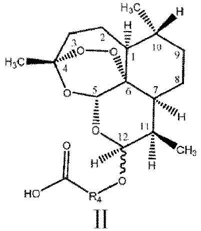
wherein R₄ is -(CH₂)_{y}, -CO(CH₂)_{y}, -C₆H₆(CH2)_{y} or -COC₆H₆(CH₂)_{y}, wherein y is an integer greater than or equal to 0, CO is a carbonyl and C₆H₆ is a benzene ring.

2. The cyclodextrin carrier-based arteannuinoid compound conjugate according to claim 1, wherein the arteannuinoid compound is artesunate.

3. A method for preparing the cyclodextrin carrier-based arteannuinoid compound conjugate according to claim 1 or 2, comprising reacting the carboxyl group of the arteannuinoid compound with the hydroxyl group of the cyclodextrin in a polar organic solvent to form an ester bond, thereby to obtain a cyclodextrin carrier-based arteannuinoid compound conjugate.

4. The preparing method according to claim 3, wherein the molar ratio of the arteannuinoid compound to the cyclodextrin is 10:1 to 1:15.

5. The preparing method according to claim 3 or 4, wherein the arteannuinoid compound and the cyclodextrin is dissolved in the polar organic solvent in a weight ratio of 1:5 to 1:50, and the reaction is performed under stirring at 80 to 120°C for 24 to 192 hours.

6. The preparing method according to any one of claims 3 to 5, further comprising a step of purifying the cyclodextrin carrier-based arteannuinoid compound conjugate.

## Patentansprüche

1. Cyclodextrinträgerbasiertes Arteannuinoidverbindungskonjugat, wobei die Arteannuinoidverbindung verbunden ist über eine Esterbindung, die zwischen einer Carboxylgruppe der Arteannuinoidverbindung und einer Hydroxylgruppe des Cyclodextrin verbunden ist;
wobei das Cyclodextrin ein Sulfonat einer als Formel I dargestellten Struktur ist: 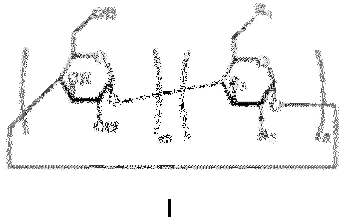
wobei folgendes gilt: n ist 1 und m+n = 6, 7 oder 8;
R₁, R₂ oder R₃ ist -ROH;
wobei R -(CH₂)ₓ, -NH(CH₂)ₓ, -NH(CH₂)ₓNH(CH₂)ₓ, -CO(CH₂)ₓ oder -O(CH₂)ₓ ist, und wobei x 0, 1, 2, 3 oder 4 ist;
wobei die Arteannuinoidverbindung eine als Formel II dargestellte Struktur aufweist 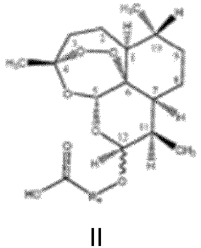
wobei R₄ -(CH₂)_{y}, -CO(CH₂)_{y}, -C₆H₆(CH₂)_{y} oder -COC₆H₆(CH₂)_{y} ist, wobei y eine ganze Zahl größer oder gleich 0 ist, wobei CO ein Carbonyl ist, und wobei C₆H₆ ein Benzolring ist.

2. Cyclodextrinträgerbasiertes Arteannuinoidverbindungskonjugat nach Anspruch 1, wobei die Arteannuinoidverbindung Artesunat ist.

3. Verfahren zur Zubereitung des cyclodextrinträgerbasierten Arteannuinoidverbindungskonjugats nach Anspruch 1 oder 2, umfassend das Reagieren der Carboxylgruppe der Arteannuinoidverbindung mit der Hydroxylgruppe des Cyclodextrin in einem polaren organischen Lösungsmittel, um eine Esterbindung zu bilden, wodurch ein cyclodextrinträgerbasiertes Arteannuinoidverbindungskonjugat erhalten wird.

4. Zubereitungsverfahren nach Anspruch 3, wobei das Molverhältnis der Arteannuinoidverbindung zu dem Cyclodextrin 10:1 bis 1:15 ist.

5. Zubereitungsverfahren nach 3 oder 4, wobei die Arteannuinoidverbindung und das Cyclodextrin in dem polaren organischen Lösungsmittel in einem Gewichtsverhältnis von 1:5 bis 1:50 aufgelöst werden, und wobei die Reaktion unter Rühren über 24 bis 192 Stunden bei 80 bis 120 °C durchgeführt wird.

6. Zubereitungsverfahren nach einem der Ansprüche 3 bis 5, das ferner einen Schritt des Aufreinigens des cyclodextrinträgerbasierten Arteannuinoidverbindungskonjugats umfasst.

## Revendications

1. Conjugué de composé d'artéannuine à base de support en cyclodextrine, dans lequel le composé d'artéannuine est connecté par une liaison ester formée entre un groupe carboxyle du composé d'artéannuine et un groupe hydroxyle de la cyclodextrine ;
dans lequel la cyclodextrine est un sulfonate d'une structure représentée en tant que formule I : 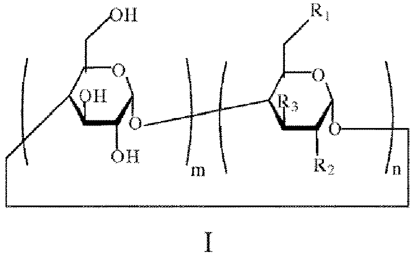
dans laquelle n vaut 1, et m+n = l'un quelconque de 6, 7 ou 8 ;
R₁, R₂ ou R₃ est -ROH ;
où R est -(CH₂)ₓ, -NH(CH₂)ₓ, -NH(CH₂)ₓNH(CH₂)ₓ, -CO(CH₂)ₓ ou -O(CH₂)ₓ, et x vaut 0, 1, 2, 3 ou 4 ;
dans lequel le composé d'artéannuine a une structure représentée en tant que formule II, 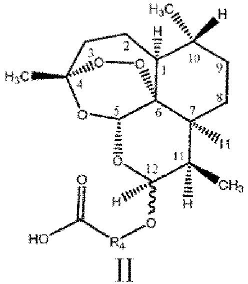
dans laquelle R₄ est -(CH₂)_{y}, -CO(CH₂)_{y}, -C₆H₆(CH₂)_{y} ou -COC₆H₆(CH₂)_{y}, où y est un entier supérieur ou égal à 0, CO est un carbonyle et C₆H₆ est un cycle benzène.

2. Conjugué de composé d'artéannuine à base de support en cyclodextrine selon la revendication 1, dans lequel le composé d'artéannuine est l'artésunate.

3. Procédé de préparation du conjugué de composé d'artéannuine à base de support en cyclodextrine selon la revendication 1 ou 2, comprenant la réaction du groupe carboxyle du composé d'artéannuine avec le groupe hydroxyle de la cyclodextrine dans un solvant organique polaire pour former une liaison ester, ce qui permet d'obtenir un conjugué de composé d'artéannuine à base de support en cyclodextrine.

4. Procédé de préparation selon la revendication 3, dans lequel le rapport molaire du composé d'artéannuine à la cyclodextrine est de 10:1 à 1:15.

5. Procédé de préparation selon la revendication 3 ou 4, dans lequel le composé d'artéannuine et la cyclodextrine sont dissous dans le solvant organique polaire en un rapport en poids de 1:5 à 1:50, et la réaction est réalisée sous agitation à 80 à 120 °C pendant 24 à 192 heures.

6. Procédé de préparation selon l'une quelconque des revendications 3 à 5, comprenant en outre une étape de purification du conjugué de composé d'artéannuine à base de support en cyclodextrine.
